# EUROPEAN PATENT APPLICATION

(11) **EP 0 920 860 A2**
(43) Date of publication of application: **09.06.1999**
(21) Application number: 98122054.4
(22) Date of filing: 20.11.1998
(51) Int. Cl.: A61K 7/46, A61K 7/32, A01N 25/04

(54) **Slow-release high-viscous liquid composition for a volatile substance**

(30) Priority: 28.11.1997 JP 32767497
(71) Applicant: SHOWA DENKO KABUSHIKI KAISHA, Tokyo (JP)
(72) Inventor: Yamaguchi, Tetsuhiko c/o Showa Denko K.K., Kawasaki-shi, Kanagawa-ken (JP); Tagoshi, Hirotaka c/o Showa Denko K.K., Central, Chiba-shi Chiba-ken (JP); Wada, Tetsuo Showa Denko K.K.,Kawasaki Res.Lab., Kawasaki-shi, Kanagawa-ken (JP)
(74) Representative: Strehl Schübel-Hopf & Partner

(57) **Abstract**

To provide a slow-release high-viscous liquid composition for a volatile substance, which can contain a surfactant or alcohol for dispersion solubilizing the volatile substance in a high concentration and has excellent light stability. A slow-release high-viscous liquid composition for a volatile substance, comprising a volatile substance, poly-N-vinylcarboxylic acid amide-base resin soluble in water or hydrous alcohol, a surfactant, water and if desired, an alcohol.

## Description

### BACKGROUND OF THE INVENTION

### Field of the Invention

The present invention relates to a slow-release high-viscous liquid composition for a volatile substance used in public facilities such as hospital, indoor facilities such as housing, toilet, dressing room, shoe box, chest of drawers, closet, garret and underfloor part, vehicles such as car, train and airplane, and outdoor facilities such as garden, rooftop, street, garbage pit, compost box, orchard and hothouse.

This application is based on patent application No. Hei 9-327674 filed in Japan and provisional patent application serial No. 60/077106 filed in U.S., the contents of which are incorporated herein by reference.

### Description of Related Art

Conventionally, perfume, deodorants, insectifuges, pesticides, repellents and the like are a liquid product in many cases and accordingly, these are held in a bottle and on use, the bottle is opened so that the volatile substance as an active ingredient can evaporate or the liquid can be sprayed or atomized. However, when the liquid product held in a bottle in the open state is left standing, the bottle may fall down to cause contamination in the surrounding or the effect disadvantageously does not continue due to its high evaporation rate.

In order to solve these problems, a method of adsorbing a volatile substance to an inorganic porous carrier such as silica, or to a spongy resin carrier and a method of kneading a volatile substance into a resin to immobilize the substance have been developed. Further, a gel formation method using a gelling agent which is a natural polymer compound such as carrageenan, sodium alginate, gellan gum, agar, gelatin and chitosan, or a synthetic polymer compound such as urethane-base compound, vinyl alcohol-base compound and acryl-base compound, is used.

By using these methods, the problem of splashing of the contents on falling down of the container is solved. However, in the case of using a carrier or kneaded resin, the amount of the volatile ingredient contained is limited and further, the volatile ingredient contained inside is restricted in the evaporation to cause a problem in view of the duration of slow release property with the passage of time. The natural polymer compound such as carrageenan and agar is once dissolved under heating at a high temperature and gelled under cooling to form a gel composition and since the gelation temperature is high, for example, in the case of use as a gel perfume, the perfume as a volatile substance must be added at a temperature higher than the gelation temperature. As a result, a part of the perfume ingredient volatilizes to present a balance different from the original perfume ingredient, which gives rise to a problem of uncomfortable feeling not favorable to the perfume. Furthermore, a solution containing a large amount of a volatile ingredient solubilizing agent such as a surfactant or alcohol, is deficient in that it cannot be gelled. Therefore, a problem is present that a solution containing a volatile ingredient in a high concentration cannot be gelled. Also, deterioration due to putrefaction with the passage of time, generation of mold and a problem in view of weather resistance still remain to be solved.

On the other hand, a method of thickening a liquid containing a volatile ingredient is used to solve the problem of contamination due to leakage of the liquid or the problem in view of durability. This method is advantageous in that the thickener can be used without heating and due to the presence of flowability unlike prepared by using the method of immobilization or gelation, the thickened solution is easily transported or filled in a container.

The thickener includes synthetic thickeners such as polyvinyl alcohol, polyethylene glycol, polyethylene oxide, polyacrylic acid or a metal salt thereof, polyacrylamide and polyvinyl pyrrolidone, and natural thickeners such as carboxymethyl cellulose, hydroxyalkyl cellulose and methyl cellulose. However, also in the case of using a thickener, the liquid containing an alcohol or a surfactant in a high concentration may not be thickened or extremely low in the stability against the ultraviolet ray, moreover, the natural thickener has a problem such as putrefaction or generation of mold.

JP-A-9-66095 (the term "JP-A" as used herein means an "unexamined published Japanese patent application") discloses a perfume in the state of liquid to gel using a cationic thickener, but this has a problem such that since the thickener is cationic, when the volatile substance, surfactant or additive used is anionic or cationic, the effect or the slow-release property is inhibited.

### SUMMARY OF THE INVENTION

As a result of extensive studies to solve the above-described problems, the present invention provides a slow-release high-viscous liquid composition for a volatile substance, which can contain a surfactant or alcohol for dispersion solubilizing the volatile substance in a high concentration and has excellent light stability.

The present invention relates to the following matters:
(1) A slow-release high-viscous liquid composition for a volatile substance, comprising a volatile substance, a poly-N-vinylcarboxylic acid amide-base resin soluble in water or hydrous alcohol, a surfactant and water.
(2) The slow-release high-viscous liquid composition for a volatile substance as described in 1 above, which contains an alcohol.
(3) The slow-release high-viscous liquid composition for a volatile substance as described in 1 or 2 above, wherein the volatile substance is selected from the group consisting of perfumes, deodorants, insectifuges, pesticides and repellents.
(4) The slow-release high-viscous liquid composition for a volatile substance as described in any of 1 to 3 above, wherein the poly-N-vinylcarboxylic acid amide-base resin is selected from the group consisting of poly-N-vinylformamide-base resins, poly-N-vinylacetamide-base resins, poly-N-methyl-N-vinylacetamide-base resins and poly-N-vinyl-α-pyrrolidone-base resins.
(5) The slow-release high-viscous liquid composition for a volatile substance as described in 4 above, wherein the poly-N-vinylcarboxylic acid amide-base resin is a poly-N-vinylacetamide-base resin.
(6) The slow-release high-viscous liquid composition for a volatile substance as described in 5 above, wherein the poly-N-vinylacetamide-base resin is an N-vinylacetamide-base homopolymer.
(7) The slow-release high-viscous liquid composition for a volatile substance as described in any of 1 to 6, wherein said poly-N-vinylcarboxylic acid amide-base resin has such a property that it can be homogeneously dissolved in water or hydrous alcohol within 24 hours when said poly-N-vinylcarboxylic acid amide-base resin is added to water or hydrous alcohol in an amount of from 0.1% to 6% by weight.
(8) The slow-release high-viscous liquid composition for a volatile substance as described in any of 1 to 7, which has viscosity of 10,000 cps or more.
(9) Use of a high-viscous liquid composition for slow release of a volatile substance, said high-viscous liquid composition comprises a volatile substance, a poly-N-vinylcarboxylic acid amide-base resin soluble in water or hydrous alcohol, a surfactant and water.
(10) Use of a high-viscous liquid composition for slow release of a volatile substance as described in 9 above, wherein said volatile substance is selected from the group consisting of perfumes and deodorants.

The present invention provides a slow-release high-viscous liquid composition for a volatile substance, which can contain a surfactant or alcohol for dispersion solubilizing the volatile substance in a high concentration and has excellent light stability. Furthermore, the slow-release high-viscous liquid composition for a volatile substance can be advantageous in that due to the presence of flowability, unlike when prepared using a method of immobilization or gelation, the thickened solution is easily transported or filled into a container. Additionally, by agitating the flowable slow-release high-viscous liquid composition for a volatile substance, the slow-release property can easily be restored.

### DESCRIPTION OF THE PREFERRED EMBODIMENTS

The present invention is described in detail below.

The poly-N-vinylcarboxylic acid amide-base resin for use in the present invention is a soluble polymer obtained by polymerizing an N-vinylcarboxylic acid amide alone such as N-vinylformamide, N-vinylacetamide, N-methyl-N-vinylacetamide or N-vinyl-α-pyrrolidone, or polymerizing the N-vinylcarboxylic acid amide with monomer copolymerizable therewith.

The term "soluble" as used herein means that the polymer, that is, the poly-N-vinylcarboxylic acid amide-base resin, can be dissolved in water or hydrous alcohol and when the polymer is added to water or hydrous alcohol in an amount of from 0.1% to 6% by weight, the polymer can be homogeneously dissolved within 24 hours to give a transparent solution. The term "hydrous alcohol" as used herein means a hydrous alcohol containing either one (two or more may also be used in combination) of a monohydric alcohol such as methyl alcohol, ethyl alcohol, propyl alcohol, isopropyl alcohol, butyl alcohol, isobutyl alcohol, pentanol and benzyl alcohol, and a polyhydric alcohol such as ethylene glycol, propylene glycol, 1,3-butylene glycol, 1,4-butylene glycol, diethylene glycol, triethylene glycol and glycerin, in an amount of 70% by weight or less.

The poly-N-vinylcarboxylic acid amide-base resin for use in the present invention is preferably a poly-N-vinylacetamide-base resin and in view of aging stability and alcohol thickening property, more preferably an N-vinylacetamide homopolymer.

Examples of the monomer copolymerized with the N-vinylcarboxylic acid amide include (meth)acrylic acid-base monomers such as (meth)acrylic acid (and alkali metal salt or ammonium salt thereof), methyl (meth)acrylate, isopropyl (meth)acrylate, butyl (meth)acrylate, octyl (meth)acrylate, 2-ethylhexyl (meth)acrylate, methoxyethyl (meth)acrylate, 2-hydroxyethyl (meth)acrylate, 2-hydroxypropyl (meth)acrylate and polyoxyalkylene glycol mono(meth)acrylate; (meth)acrylamide-base monomers such as (meth)acrylamide, N,N-dimethyl(meth)acrylamide, N,N-diethyl(meth)acrylamide, N-methylol(meth)acrylamide, (meth)acrylamide-2-methylsulfonic acid (and alkali metal salt or ammonium salt thereof) and N-isopropyl(meth)acrylamide; vinyl ester-base monomers such as vinyl acetate, vinyl butyrate and vinyl valerate; styrene-base monomers such as styrene, α-methylstyrene, *p*-methylstyrene, *p*-methoxystyrene, *m*-chlorostyrene and vinylpyridine; vinyl ether-base monomers such as methyl vinyl ether, butyl vinyl ether and vinyl benzyl ether; dicarboxylic acid-base monomers such as maleic anhydride, maleic acid (and alkali metal salt or ammonium salt thereof), fumaric acid (and alkali metal salt or ammonium salt thereof), maleic acid dimethyl ester and fumaric acid diethyl ester; allyl-base monomers such as allyl alcohol, allyl phenyl ether and allyl acetate; and monomers such as (meth)acrylonitrile, vinyl chloride, vinylpyrrolidone and N-vinylimidazole.

The ammonium salt includes a salt of ammonia, alkylamines such as triethylamine, diethylamine and dibutylmonomethylamine, OH group-containing organic amines such as monoethanolamine, diethanolamine, triethanolamine, monoisopropanolamine, N-methylethanolamine and dimethylaminopropanolamine, and cyclic organic amines such as morpholine.

Two or more of the above-described monomers may be copolymerized in combination.

The ratio of the copolymerizable monomer used is suitably in a range of not impairing the object and capability of the present invention and it is generally 60% by weight or less, preferably 40% by weight or less.

By using a poly-N-vinylcarboxylic acid amide-base resin, even a solution containing a surfactant or alcohol for dispersion solubilizing a volatile substance can be easily thickened. Further, the high-viscous liquid composition obtained has excellent light stability and is difficultly reduced in the viscosity with the passage of time during storage or use for a long period of time.

The amount of poly-N-vinylcarboxylic acid amide-base resin added varies depending on the molecular weight of the polymer or the viscosity required for the slow-release high-viscous liquid composition for a volatile substance, but it is usually from 0.1% to 30% by weight, preferably from 0.5% to 20% by weight. If the amount used is less than 0.1% by weight, sufficiently high viscosity cannot be obtained in many cases, whereas if it exceeds 30% by weight, the viscosity is too high and this is disadvantageous in that the workability in filling the composition in a container is bad and the residual amount after use and volatilization of the volatile substance tends to be large.

The poly-N-vinylcarboxylic acid amide-base resin is produced by the solution polymerization method, the reversed phase suspension polymerization method or the precipitation deposition polymerization method, which are all known.

The volatile substance for use in the present invention is a substance capable of evaporation at room temperature, such as perfume, deodorant, insectifuge, pesticide and repellent, and one or more thereof are used. The amount of the volatile substance used may be appropriately selected depending on the kind, use end and form of the slow-release high-viscous liquid composition for a volatile substance, but it is generally from 0.1% to 60% by weight, preferably from 0.5% to 50% weight, based on the entire amount. If the amount used is less than 0.1% by weight, the objective functions of the volatile substance-containing composition cannot be brought out in many cases, whereas if it exceeds 60% by weight, the volatile substance excessively volatilizes and the effect is disadvantageously liable to be too much intensified.

In the case of the slow-release high-viscous liquid composition for a volatile substance according to the present invention, the composition can be thickened even if the content of the volatile substance increases, as compared with the case of using a conventional thickener. Accordingly, a volatile substance-containing high-viscous liquid composition having a high concentration can be produced, as a result, the product manufactured can be more reduced in the size.

Examples of the perfume for use in the present invention as the volatile substance include a flavor of citrus such as orange, lemon, lime and grapefruit; a flavor of fruits such as pineapple, banana, apple, apricot, berry, cherry and peach; a flavor for food, such as coconut, vanilla and mint; a flavor of flowers such as rose, jasmine, fragrant olive, gardenia, lily of the valley, sweet pea, lilac, freesia, hyacinth and cyclamen; hydrocarbons such as limonene, α-pinene, β-pinene, *p*-cymene, γ-terpinene, 3-carene, myrcene, ocimene, *p*-menthane and 1-*p*-menthene; alcohols and alcohol esters thereof, such as linalool, citronellol, geraniol, benzyl alcohol and β-phenylethyl alcohol; perfume compounds conventionally used as the material for mixed perfumes; and mixed perfume compositions comprising an optional mixture of these flavors or compounds.

Examples of the deodorant for use as the volatile substance include plant extracted essential oil, dithio-2,2'-bis(benzomethylamide) and 1,2-benzisothiazolin-3-one.

Examples of the insectifuge for use as the volatile substance include permethrine, prothiofos, fluvalinate, fenvalerate, pyrethrin, piperonyl butoxide, camphor oil, lavender oil, capsicum extract and garlic extract.

Examples of the pesticide for use as the volatile substance include chloropicrin, dichloropropane, dichloropropylene, dichlorvos and liquid drugs commonly used as the agricultural chemical at present.

Examples of the repellent for use as the volatile substance include repellents for vermin, cats and dogs, crows, hares and the like, such as fenvalerate, lemongrass essential oil, dry distilled wood essence and tobacco extract.

The surfactant for use in the present invention is not particularly limited as far as it can dispersion solubilize the volatile substance, and nonionic, anionic and cationic surfactants may be used.

Examples of the surfactants for use in the present invention include polyoxyethylene derivatives such as polyoxyethylene alkyl ethers, polyoxyethylene fatty acid esters, polyoxyethylene fatty acid amides and polyoxyethylene hydrogenated castor oils, polyoxyethylene polyoxypropylene copolymers, sorbitan fatty acid esters, polyoxyethylene sorbitan fatty acid esters, sorbitol derivatives, sucrose fatty acid esters, fatty acid salts, alkylsulfuric acid esters, alkylbenzenesulfonates, alkylnaphthalenesulfonates, alkylsulfosuccinates, alkyldiphenyl ether disulfonates, polyoxyethylene alkylsulfonic acid ester salts, alkylamine salts, quaternary ammonium salts, alkylbetaines, amine oxides, and any mixture thereof.

The amount of the surfactant mixed is selected to be an optimal amount for solubilizing the volatile substance, but it is usually from 0.1% to 20% by weight, preferably from 0.5% to 15% by weight, based on the entire amount of the high-viscous liquid composition. If the amount mixed exceeds 20% by weight, volatilization of the volatile substance from the high-viscous liquid composition is disadvantageously very liable to be inhibited.

The amount of water mixed in the present invention may be appropriately selected according to the form on use of the slow-release high-viscous liquid composition for a volatile substance, but it is generally from 15% to 99.8% by weight, preferably from 30% to 98% by weight, based on the entire amount of the high-viscous liquid composition. If the amount is less than this range or exceeds this range, the effect of thickening the solution containing a volatile substance cannot be attained in many cases and this is not preferred.

In the present invention, an alcohol is not necessarily mixed but may be mixed for the purpose of freeze proofing in the severe cold season and controlling the volatilization rate of the volatile substance, or further for the purpose of improving the transparency of the high-viscous liquid composition. The mixing amount thereof may be freely selected, but it is generally from 0% to 90% by weight based the entire amount of the high-viscous liquid composition.

Examples of the alcohols for use in the present invention include methyl alcohol, ethyl alcohol, isopropyl alcohol, benzyl alcohol and 3-methyl-3-methoxybutanol, and in view of safety, ethyl alcohol is preferred. Modified alcohols having added thereto a modifier of various types, such as octaacetylated sucrose, denatonium benzoate, brucine, Flavor H-No. 1, Flavor H-No. 3, Flavor H-No. 4, Flavor H-No. 6, Flavor H-No. 9, Flavor H-No. 10, Flavor H-No. 11, Flavor H-No. 12, Flavor H-No. 13, Flavor T-No. 100 and Flavor T-No. 101, may also be used. Further, polyhydric alcohols such as ethylene glycol, diethylene glycol, triethylene glycol, propylene glycol, 1,3-butyl glycol, hexylene glycol, glycerin, sorbitol and maltitol, may be used individually or in combination.

If desired, the slow-release high-viscous liquid composition for a volatile substance of the present invention may contain, in addition to the above-described essential ingredients, a dye, an ultraviolet absorbent, an antiseptic, a bitter agent, a thickener, an antioxidant and a conventional gelling agent.

The dye is used for the purpose of coloring the high-viscous liquid composition and thereby improving the design, and examples thereof include Brilliant Blue FCF, Acid Red, Erythrosine and Sunset Yellow FCF. The ultraviolet absorbent is added when the volatile substance contained in the slow-release high-viscous liquid composition for a volatile substance is easy to deteriorate due to the ultraviolet ray, and examples thereof include hydroxymethoxybenzophenone, phenyl salicylate, benzotriazole-base absorbents and cyanoacrylate-base absorbents. Examples of the antiseptic include para-hydroxybenzoic acid ester and isopropylmethylphenol, and the addition amount thereof can be greatly reduced than usual because the poly-N-vinylcarboxylic acid amide-base resin has excellent putrefaction resistance. The bitter agent is added to the high-viscous liquid composition so as to prevent wrong eating by infants and the like, and examples thereof include BITLEX (registered trademark: sold by Nagase Sangyo). The antioxidant is added mainly for the purpose of preventing oxidation deterioration of the volatile substance, and examples thereof include vitamin E, di-t-butyl-*p*-cresol (BHT) and butylated hydroxyanisole (BHA).

In the case of mixing these additives, the objective functions thereof can be sufficiently brought out when they are each added in an amount of approximately from 0% to 2% by weight based on the entire amount of the high-viscous liquid composition.

The slow-release high-viscous liquid composition for a volatile substance of the present invention can be produced, for example, through the following simple operation. A volatile substance is poured into water or an aqueous alcohol solution, a surfactant is added thereto in an appropriate amount and the mixture is stirred to homogeneously dissolve or disperse the volatile substance. Then, poly-N-vinylcarboxylic acid amide-base resin is poured thereinto and the mixture is stirred at room temperature for several hours. The time required for bringing out the thickening effect varies depending on the composition of the solution, but it is generally on the order of from 1 to a few hours. Or, a volatile substance-containing solution (dispersion solution) is prepared and then poured into a desired product container where poly-N-vinylcarboxylic acid amide-base resin is previously placed, to obtain a high-viscous liquid composition.

### Examples

The present invention is described below by referring to the Examples and Comparative Examples, however, it is needless to say that the present invention is not limited thereto.

### Examples 1 to 16

Slow-release high-viscous liquid compositions for a volatile substance were prepared according to the mixing amount (% by weight) shown in Tables 1 and 2 below. Examples 1 to 10 and 15 to 16 are the formulation examples for the perfume, Example 11 for the deodorant, Example 12 for the insectifuge, Example 13 for the pesticide and Example 14 for the repellent. Of the ingredients, the perfume (muscat, peach and vanilla flavors), insectifuge (lavender oil) and repellent (lemongrass essential oil) used were commercially available products. The surfactant used was a commercially available product produced by Kao Corporation. The poly-N-vinylcarboxylic acid amide-base resin used was a homopolymer of N-vinylacetamide, poly-N-vinylacetamide resin "PNVA GE-191", produced by Showa Denko KK (1% aqueous solution viscosity: 200 cps; viscosity measurement conditions: BM type rotational viscometer, 20°C, revolution number: 30 rpm), a copolymer resin of N-vinylacetamide and sodium acrylate, "PNVA GE-167" produced by Showa Denko KK (1% aqueous solution viscosity: 4,000 cps; viscosity measurement conditions: BM type rotational viscometer, 20°C, revolution number: 30 rpm), or a copolymer resin of N-vinylacetamide and 2-hydroxyethyl methacrylate (monomer weight ratio: 7/3) (1% aqueous solution viscosity: 400 cps; viscosity measurement conditions: BM type rotational viscometer, 20°C, revolution number: 30 rpm).

The preparation method was such that a volatile substance was poured into water or an aqueous alcohol solution, a surfactant was added thereto in an appropriate amount, the mixture was stirred to homogeneously dissolve or disperse the volatile substance and then, poly-N-vinylcarboxylic acid amide-base resin was added to the resulting solution or dispersion solution while stirring.

**Table 1**

| Ingredient | Example | | | | | | | |
|---|---|---|---|---|---|---|---|---|
| | 1 | 2 | 3 | 4 | 5 | 6 | 7 | 8 |
| Perfume | | | | | | | | |
| Muscat | 10.0 | 30.0 | 5.0 | | | | | |
| Peach | | | | 5.0 | 10.0 | 20.0 | 20.0 | |
| Vanilla | | | | | | | | 20.0 |
| Surfactant | | | | | | | | |
| EMULGEN 909 | 5.0 | 5.0 | 3.0 | 5.0 | | | | 5.0 |
| EMULGEN 903 | | | | | | | 5.0 | |
| NEOPELEX F65 | | | | | 5.0 | | | |
| AMEETO 320 | | | | | | 5.0 | | |
| Ion exchange water | 79.99 | 51.7 | 53.9 | 85.3 | 80.5 | 70.5 | 35.5 | 35.5 |
| Ethanol | | | 35.0 | | | | 35.0 | 35.0 |
| | | | | | | | | |
| PNVA GE-191 | 5.0 | 4.0 | 3.0 | 4.5 | 4.5 | 4.5 | 4.5 | 4.5 |
| | | | | | | | | |
| Glycerin | | 9.0 | | | | | | |
| Brilliant Blue FCF | 0.01 | | | | | | | |
| Phenyl salicylate | | | 0.1 | | | | | |
| BHT | | | | 0.2 | | | | |
| Methylparaben | | 0.3 | | | | | | |
| (Note): The numerical values of the ingredients mixed are all % by weight. | | | | | | | | |

**Table 2**

| Ingredient | Example | | | | | | | |
|---|---|---|---|---|---|---|---|---|
| | 9 | 10 | 11 | 12 | 13 | 14 | 15 | 16 |
| Perfume Vanilla | 10.0 | 9.0 | | | | | 5.0 | 10.0 |
| Deodorant Dithio-2,2'-bis-(benzomethylamide) | | | 0.3 | | | | | |
| Insectifuge Lavender oil | | | | 10.0 | | | | |
| Pesticide Chloropicrin | | | | | 10.0 | | | |
| Repellent Lemongrass essential oil | | | | | | 10.0 | | |
| Surfactant EMULGEN 909 | 5.0 | 4.0 | 1.0 | 5.0 | 5.0 | 5.0 | 3.0 | 5.0 |
| Ion exchange water | 40.0 | 74.0 | 50.0 | 41.0 | 24.0 | 40.0 | 54.0 | 40.0 |
| Ethanol | 40.0 | | 46.7 | 39.0 | 55.0 | 40.0 | 34.5 | 40.0 |
| | | | | | | | | |
| PNVA GE-191 | 5.0 | 4.0 | 2.0 | 5.0 | 6.0 | 5.0 | | |
| PNVA GE-167 | | | | | | | 3.5 | |
| NVA/HEMA Copolymer | | | | | | | | 5.0 |
| | | | | | | | | |
| PEG #400 | | 9.0 | | | | | | |
| (Notes): The numerical values of the ingredients mixed are all % by weight. NVA/HEMA copolymer is a copolymer of N-vinylacetamide and 2-hydroxyethyl methacrylate. PEG #400 is polyethylene glycol having an average molecular weight of 400. | | | | | | | | |

### Comparative Examples 1 to 3

High-viscous liquid compositions were prepared using a commercially available natural thickener, hydroxyethyl cellulose (1% aqueous solution viscosity: 500 cps; viscosity measurement conditions: BM type rotational viscometer, 20°C, revolution number: 30 rpm) according to the mixing amount (% by weight) shown in Table 3 below. The preparation method was such that hydroxyethyl cellulose was added to water and dissolved, a perfume, a surfactant and ethanol were added thereto and the mixture was stirred to prepare a high-viscous liquid composition. Comparative Examples 4 to 7

High-viscous liquid compositions were prepared according to the mixing amount (% by weight) shown in Table 3 below in the same manner as in Comparative Example 1 except for using a commercially available sodium polyacrylate (PAS)-base thickener (1% aqueous solution viscosity: 200 cps; viscosity measurement conditions: BM type rotational viscometer, 20°C, revolution number: 30 rpm).

**Table 3**

| Ingredient | Comparative Example | | | | | | |
|---|---|---|---|---|---|---|---|
| | 1 | 2 | 3 | 4 | 5 | 6 | 7 |
| Perfume | | | | | | | |
| Muscat | | | 20.0 | 5.0 | 20.0 | 10.0 | 10.0 |
| Vanilla | 2.5 | 2.5 | | | | | |
| Surfactant | | | | | | | |
| EMULGEN 909 | 2.5 | 2.5 | 5.0 | 5.0 | 5.0 | | |
| NEOPELEX F65 | | | | | | 5.0 | |
| AMEET 320 | | | | | | | 5.0 |
| Ion exchange water | 46.5 | 68.0 | 72.0 | 44.0 | 70.0 | 83.0 | 80.0 |
| Ethanol | 46.5 | 25.0 | | 44.0 | | | |
| | | | | | | | |
| Hydroxyethyl cellulose | 2.0 | 2.0 | 3.0 | | | | |
| PAS-base thickener | | | | 2.0 | 5.0 | 2.0 | 5.0 |
| (Notes): The numerical values of the ingredients mixed are all % by weight. | | | | | | | |

### Test Example

High-viscous liquid compositions obtained above were examined on the state of viscousness and transparency at the preparation and the aging stability (reduction in the transparency, change in the viscosity, separation) during storage under sealing. The test method is described below.

### State of Viscousness:

The state of viscousness was measured using a BM type rotational viscometer under conditions of 20°C and a revolution number of 12 rpm. The evaluation was made according to the criteria: "high-viscous liquid of 10,000 cps or more" is ⓞ, "viscous liquid of 5,000 to 10,000 cps having somewhat high flowability" is ○, "viscous liquid of 5,000 cps or more but with separation" is △ and "low-viscous liquid of less than 5,000 cps" is X.

### Transparency:

The transparency was visually observed. The evaluation was made according to the criteria: "good" is ⓞ, "good but with turbidity" is ○, "somewhat opaque" is △ and "opaque" is X.

### Separation:

After one-month storage indoors under sealing at room temperature in a bright place free of direct sunlight, the degree of separation was visually examined. The evaluation was made according to the criteria: "no separation" is ⓞ, "slight separation" is ○, "some separation" is △ and "considerable separation" is X.

The test results are shown in Tables 4 and 5.

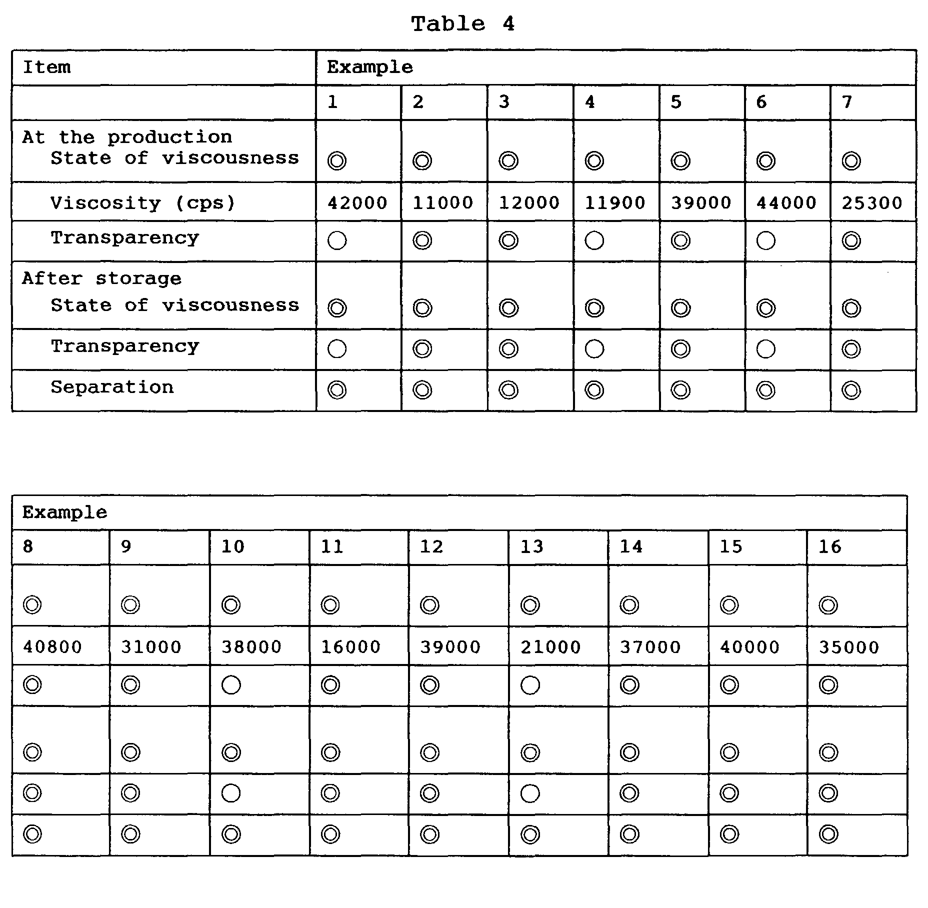

**Table 5**

| Item | Comparative Example | | | | | | |
|---|---|---|---|---|---|---|---|
| | 1 | 2 | 3 | 4 | 5 | 6 | 7 |
| At the production | | | | | | | |
| State of viscousness | △ | ⓞ | ⓞ | X | ⓞ | ○ | △ |
| Viscosity (cps) | 7000 | 26000 | 36000 | - | 36000 | 8000 | 9500 |
| Transparency | - | ○ | △ | - | X | ○ | △ |

| After storage | | | | | | | |
|---|---|---|---|---|---|---|---|
| State of viscousness | - | △ | △ | - | X | X | X |
| Transparency | - | ○ | △ | - | X | △ | △ |
| Separation | - | △ | △ | - | △ | △ | △ |

As apparent from Table 4, the high-viscous liquid compositions of Examples 1 to 16 each exhibited excellent properties in all items. On the other hand, as shown in Table 5, the composition of Comparative Example 1 had no compatibility between the aqueous ethanol solution and the aqueous hydroxyethyl cellulose solution and the solutions were separated. In Comparative Examples 2 and 3, although a high-viscous composition was obtained, the compositions were deficient in the stability against aging and separation was observed. In Comparative Example 4, the sodium polyacrylate-base thickener had almost no thickening effect on the aqueous ethanol solution and a high-viscous composition could not be obtained. In Comparative Example 5, the high-viscous liquid had no transparency and had poor aging stability. In Comparative Examples 6 and 7, the solutions were poor in the stability and partly had separation with the passage of time.

## Claims

1. A slow-release highly viscous liquid composition for a volatile substance, comprising a volatile substance, a poly-N-vinylcarboxylic acid amide-base resin soluble in water or hydrous alcohol, a surfactant and water.

2. A slow-release highly viscous liquid composition for a volatile substance as claimed in Claim 1, which contains an alcohol.

3. A slow-release highly viscous liquid composition for a volatile substance as claimed in Claim 1 or 2, wherein said volatile substance is selected from the group consisting of perfumes, deodorants, insectifuges, pesticides and repellents.

4. A slow-release highly viscous liquid composition for a volatile substance as claimed in any of Claims 1 to 3, wherein said poly-N-vinylcarboxylic acid amide-base resin is selected from the group consisting of poly-N-vinylformamide-base resins, poly-N-vinylacetamide-base resins, poly-N-methyl-N-vinylacetamide-base resins, poly-N-vinyl-α-pyrrolidone-base resins.

5. A slow-release highly viscous liquid composition for a volatile substance as claimed in Claim 4, wherein said poly-N-vinylcarboxylic acid amide-base resin is a poly-N-vinylacetamide-base resin.

6. A slow-release highly viscous liquid composition for a volatile substance as claimed in Claim 5, wherein said poly-N-vinylacetamide-base resin is an N-vinylacetamide-base homopolymer.

7. A slow-release highly viscous liquid composition for a volatile substance as claimed in any of Claims 1 to 6, wherein said poly-N-vinylcarboxylic acid amide-base resin has the property of being homogeneously dissolved in water or hydrous alcohol within 24 hours when said poly-N-vinylcarboxylic acid amide-base resin is added to water or hydrous alcohol in an amount of from 0.1% to 6% by weight.

8. A slow-release highly viscous liquid composition for a volatile substance as claimed in any of Claims 1 to 7, which has viscosity of 10,000 cps or more.

9. Use of a highly viscous liquid composition for slow release of a volatile substance, said highly viscous liquid composition being as defined in any of the claims 1 to 8.

10. Use of a highly viscous liquid composition for slow release of a volatile substance as claimed in Claim 9, wherein said volatile substance is selected from the group consisting of perfumes and deodorants.
